Europäisches Patentamt

European Patent Office

Office européen des brevets

(19)

(11) Publication number: **0 026 507**
**B1**

(12)

# EUROPEAN PATENT SPECIFICATION

(45) Date of publication of patent specification: 23.03.83

(21) Application number: 80200796.3

(22) Date of filing: 23.08.80

(51) Int. Cl.³: **C 07 C 51/265,**
**C 07 C 63/06**

(54) Process for preparing benzoic acid.

(30) Priority: 01.09.79 NL 7906575

(43) Date of publication of application:
08.04.81 Bulletin 81/14

(45) Publication of the grant of the patent:
23.03.83 Bulletin 83/12

(84) Designated Contracting States:
BE DE FR GB IT NL

(56) References cited:
GB - A - 1 063 964
GB - A - 1 467 886

(73) Proprietor: STAMICARBON B.V.
Postbus 10
NL-6160 MC Geleen (NL)

(72) Inventor: Jongsma, Cornelis
Drossaertweide 1
NL-6438 HS Oirsbeek (NL)
Inventor: Laugs, Wilhelmus Th.A.M.
Keerenderkerkweg 27
NL-6171 VK Stein (L.) (NL)

(74) Representative: Hoogstraten, Willem Cornelis
Roeland et al,
OCTROOIBUREAU DSM Postbus 9
NL-6160 MA Geleen (NL)

Courier Press, Leamington Spa, England.

Process for preparing benzoic acid

The invention relates to a process for preparing benzoic acid by oxidation of toluene in the liquid phase in the absence of an aliphatic carboxylic acid, by means of a gas containing molecular oxygen and in the presence of a catalyst consisting of a cobalt compound that is soluble in the reaction mixture. A process of this type is known from 'Hydrocarbon Processing' *13*, 11, 173—176 (1964).

This process has the drawback that, in order to attain an economic degree of conversion, it must be effected at rather high temperature, which results in a rather low selectivity of the desired reaction.

The invention aims at improvement in this respect. The process according to the invention is characterized by the fact that in addition to the cobalt compound a catalyst is used that consists of a zirconium and/or hafnium compound soluble in the reaction mixture. For convenience, the term zirconium will be used exclusively hereinafter, but in all cases where it occurs it can be replaced by hafnium or zirconium-hafnium mixtures.

The use of zirconium as a catalyst in addition to cobalt enables a higher reaction rate to be reached at the same temperature, or the reaction to be carried out at a lower temperature with the same reaction rate, with a considerably better selectivity for the desired benzoic acid being obtained at the same reaction rate than if only cobalt is used under otherwise identical conditions.

A great many atomic ratios between zirconium and cobalt may be used in the process according to the invention. Particularly suitable atomic ratios of zirconium to cobalt are ratios lower than 1:5, preferably lower than 1:10.

The zirconium and cobalt compounds used by preference are the salts of benzoic acid. The metals may also be added, for instance, in the form of the salts of aliphatic carboxylic acids with 1—20 carbon atoms, hydroxides, complex compounds, other organic salts, and alcoholates. Under the reaction conditions the salts of benzoic acid readily form from the compounds added.

It should be noted that the use of cobalt and zirconium compounds in the oxidation of toluene in an aliphatic carboxylic acid with 2—4 carbon atoms as a solvent is known from British Patent Specification 1,063,964.

This process has some drawbacks. In this process the aliphatic carboxylic acid must be removed from the reaction product, which involves extra operations. Additional costs are involved in the use of an aliphatic carboxylic acid also, for instance, in making up for that portion of the aliphatic carboxylic acid which is lost in a process step, and in regenerating the aliphatic carboxylic acid. In order to make the same amount of toluene react with oxygen for a given period of time a larger reactor volume is required than in the process according to the invention, since the required aliphatic carboxylic acid also occupies space. Since aliphatic carboxylic acids are usually stronger acids than benzoic acid, the former will give rise to corrosion problems.

In the process according to the invention, use may also be made of substances that serve as initiators or activators of the reaction, e.g. peroxides and aldehydes. Furthermore, the reaction mixture may contain as co-catalysts, in addition to the catalyzing compounds of cobalt and zirconium and/or hafnium, other metal compounds soluble in the reaction mixture, e.g. compounds of magnesium, copper, strontrium, zinc, cadmium, mercury, aluminium, lead, tin, antimony, bismuth, silver, nickel, iron, rubidium, caesium, titanium, gallium, tungsten, platinum, chromium.

The oxidation is carried out by means of a gas containing molecular oxygen. Examples of suitable gases are air, oxygen-enriched air, air diluted with nitrogen, pure oxygen, ozone, and mixtures of these gases.

Temperature and pressure are not critical, provided a liquid phase is maintained in the reaction system during the reaction, but preference is given to a temperature of between 400 K and 500 K and a pressure of between 200 kPa and 2000 kPa. Particularly suitable are temperatures of between 410 and 460 K and pressures of between 400 kPa and 800 kPa.

The process according to the invention may be effected both batch-wise and continuously.

The invention will be further elucidated with reference to the following non-restricting examples.

Examples I—II and Comparative Experiment A

A stainless-steel 3000 cm³ reactor provided with a stirrer, a gas feed into the liquid phase and a reflux cooler received 11.1 moles of toluene, 0.1 mole of benzaldehyde, cobalt octoate and zirconium octoate, the cobalt (Co) and zirconium (Zr) being present in the amounts specified in the table.

Air was passed through this mixture under the conditions indicated in the table. In all experiments the pressure was kept at 800 kPa and the stirring speed was 1400 revolutions per minute.

After the oxygen uptake had started, the reaction was continued until 500 N litres of vent gas had been obtained, while the percentage by volume of oxygen in the vent gas was controlled between 1 and 4. The reaction product was then cooled and the content of each component, viz. benzoic acid, toluene, benzaldehyde, benzyl alcohol, benzyl formiate, benzyl acetate and benzyl benzoate, was determined by chemical analysis.

As benzyl alcohol, benzyl acetate, benzyl formiate, benzyl benzoate and benzyl aldehyde are intermediate products in the oxidation of toluene to benzoic acid, they were included with the desired product, benzoic acid, in the calculation of the reaction selectivity on the basis of the toluene consumption.

### TABLE

| Example/ comp. expt. | Co weight, p.p.m. | Zr weight, p.p.m. | Rate of benzoic acid formation g/h | Selectivity, moles % | Temperature, K |
|---|---|---|---|---|---|
| I | 61 | 1.2 | 123.7 | 93.3 | 426 |
| II | 62 | 0.5 | 81.2 | 94.5 | 421 |
| A | 63 | 0 | 92.2 | 93.5 | 426 |

Although in the examples use is made of cobalt contents of about 60 parts by weight per million, cobalt contents of between 10 and 1000 parts by weight per million can very well be applied. The zirconium content is preferably at least 0.01 part by weight per million.

## Claims

1. Process for preparing benzoic acid by oxidation of toluene in the liquid phase in the absence of an aliphatic carboxylic acid, by means of a gas containing molecular oxygen and in the presence of a catalyst consisting of a cobalt compound that is soluble in the reaction mixture, this process being characterized in that in addition to the cobalt compound a catalyst is used that consists of a zirconium and/or hafnium compound soluble in the reaction mixture.

2. Process according to claim 1, characterized in that the catalysts used are the corresponding salts of benzoic acid.

3. Process according to the claim 1 or 2, characterized in that the atomic ratio of zirconium (hafnium) to cobalt is lower than 1:5.

4. Process according to claim 3, characterized in that the atomic ratio of zirconium (hafnium) to cobalt is lower than 1:10.

## Patentansprüche

1. Verfahren zur Herstellung von Benzoesäure durch Oxydation von Toluol in der Flüssigphase in Abwesenheit einer aliphatischen Carbonsäure, mit Hilfe eines molekularen Sauerstoff enthaltenden Gases und in Anwesenheit eines aus einer Kobaltverbindung bestehenden Katalysators, der im Reaktionsgemisch löslich ist, dadurch gekennzeichnet, dass als Beigabe zur Kobaltverbindung ein Katalysator verwendet wird, der aus einer Zirkonium- und/oder Hafniumverbindung besteht, welche im Reaktionsgemisch löslich ist.

2. Verfahren nach Anspruch 1, dadurch gekennzeichnet, dass die verwendeten Katalysatoren die korrespondierenden Salze der Benzoesäure sind.

3. Verfahren nach Anspruch 1 oder 2, dadurch gekennzeichnet, dass das Atomverhältnis von Zirkonium (Hafnium) zu Kobalt niedriger is als 1:5.

4. Verfahren nach Anspruch 3, dadurch gekennzeichnet, dass das Atomverhältnis von Zirkonium (Hafnium) zu Kobalt niedriger ist als 1:10.

## Revendications

1. Procédé pour la préparation d'acide benzoïque par oxydation de toluène dans la phase liquide en l'absence d'un acid carboxylique aliphatique, au moyen d'un gaz contenant de l'oxygène moléculaire et en présence d'un catalyseur constitué d'un composé du cobalt qui est soluble dans le mélange réactionnel, le procédé étant caractérisé en ce qu'en plus du composé du cobalt, on utilise un catalyseur qui est constitué d'un composé du zirconium et/ou du hafnium soluble dans le mélange réactionnel.

2. Procédé selon la revendication 1, caractérisé en ce que les catalyseurs utilisés sont les sels correspondants d'acide benzoïque.

3. Procédé selon l'une des revendications 1 et 2, caractérisé en ce que le rapport atomique du zirconium (hafnium) au cobalt est inférieur à 1:5.

4. Procédé selon la revendication 3, caractérisé en ce que le rapport atomique du zirconium (hafnium) au cobalt. est inférieur à 1:10.